# EUROPEAN PATENT APPLICATION

(11) **EP 0 524 553 A1**
(43) Date of publication of application: **27.01.1993**
(21) Application number: 92112209.9
(22) Date of filing: 17.07.1992
(51) Int. Cl.: C07K 5/06, A61K 37/64

(54) **Acylmercaptoalkanoyldipeptides, methods of preparation and their therapeutic use**

(30) Priority: 23.07.1991 FR 9109306
(71) Applicant: INSTITUT NATIONAL DE LA SANTE ET DE LA RECHERCHE MEDICALE (INSERM), 75654 Paris Cédex 13 (FR)
(72) Inventor: Fournie-Zaluski, Marie-Claude, F-75020 Paris (FR); Roques, Bernard-Pierre, F-94410 Saint Maurice (FR)
(74) Representative: Pilard, Jacques

(57) **Abstract**

This invention is directed to a compound of the formula 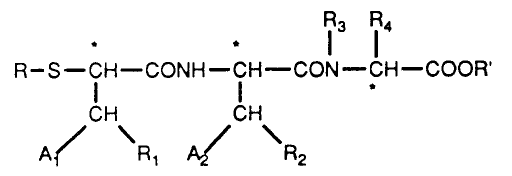
that inhibits simultaneously neutral endopeptidase and peptidyldipeptidase A and is useful in treating hypertension. The invention is also directed to the preparation of the compound, pharmaceutical compositions containing it, and methods for its pharmaceutical use.

## Description

### Field of the Invention

The present invention is directed to compounds useful as extremely potent antihypertensives and that function by the concurrent inhibition of neutral endopepti-dase and peptidyldipeptidase A. The invention is also directed to the preparation of these compounds, pharmaceutical compositions and methods for their pharmaceutical use.

### Recent Developments

European Patent EP 0,038,758 describes products which are inhibitors of neutral endopeptidase (NEP) (EC 3.4-24.11). NEP is alternatively referred to as "enkephalinase" since the enzyme degrades the enkephalins which are endogenous opiate peptides of morphine receptors. These inhibitors are consequently useful as analgesics. This reference does not disclose that NEP inhibitors possess antihypertensive activity exhibited by inhibitors of peptidyldipeptidase A (ACE) (EC 3.4-15.1).

In Nature, Vol. 288, 286-288 (1980), Roques et al. have shown that (R,S)-(2-mercaptomethyl-3-phenylpropionyl)glycine (thiorphan) has an inhibitory power at a nanomolar concentration and behaves as an analgesic in potentiating the action of the enkephalins. This reference does not disclose that thiorphan possesses antihypertensive activity for example exhibited by inhibitors of ACE.

Other inhibitors of enkephalinase, endowed with analgesic properties, are the subject, for example, of French Patent FR 83 20024 (2,556,721) and European Patent EP 0,136,883. These references do not disclose that the NEP inhibitors possess antihypertensive activity for example exhibited by inhibitors of ACE.

U.S. Patent No. 4,879,309 discloses that compounds of formulae HS-CH₂-CH(R₂)-CONH-CH(R₁)-COOR₃ and HS-(CH₂)ₘ-CH(R₂)-CONH-CH(R₁)-CO-A-COOR₃ are useful for augmenting natriuresis and diuresis which thereby aids in reducing blood pressure. This reference does not disclose that the inhibitors possess antihypertensive activity separate from their natriuretic and diuretic effects.

Koehn et al., J. Biol. Chem., 262, 11623-11627 (1987) and S. L. Stephenson and A. J. Renny, Biochem. J., 243, 183-187 (1987) have reported that auricular natriuretic peptide which is liberated by the heart, particularly in cardiac insufficiency, and which augments natriuretic, diuretic and vasodilator effects, is inactivated by the peripheral enzyme EP 24.11. Thus, the inhibitor of NEP, thiorphan, and certain of its derivatives are capable of augmenting the half-life of circulating auricular natriuretic peptide and thereby aids in reducing blood pressure in the rat. [G. Olins et al., Moll. Cell. Endocrinol., 61, 201-208 (1989); A.A. Seymour et al., Hypertension, 14, 87-97 (1989)]. However, none of these references disclose that the NEP inhibitors possess antihypertensive properties exhibited by inhibitors of ACE activity.

Furthermore, in clinical trials, the inhibitors of neutral endopeptidase, such as thiorphan, produce natriuresis and diuresis without any significant hypotensive effect, except in certain sick people showing cardiac or renal insufficiency.

U. S. Patent Nos. 4,053,651 and 4,684,660 disclose inhibitors of peptidyldipeptidase A (ACE) (EC 3.4-15.1) which are useful as antihypertensives. These reference do not disclose that the ACE inhibitors have NEP inhibitory activity.

The object of the present invention is directed to a compound that is an extremely potent antihypertensive that inhibits simultaneously NEP and ACE inhibitory properties at very low concentrations.

### SUMMARY OF THE INVENTION

The present invention is directed to a compound of the formula 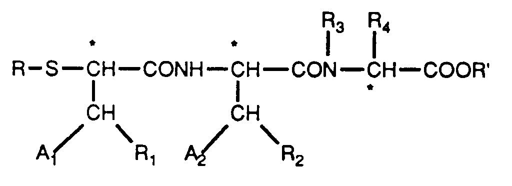
wherein
R is hydrogen, acyl, benzoyl, adamantoyl or a radical of the formula 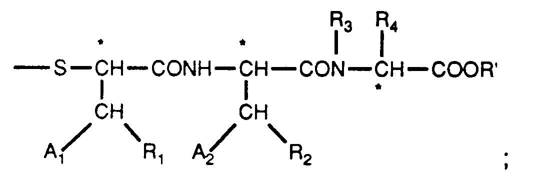
R₁ and R₂ are independently hydrogen, alkyl, phenyl, benzyl, alkoxy, alkyloxymethyl or benzyloxy;
A₁ and A₂ are independently hydrogen, methyl, or together with -CH-R₁ or -CH-R₂, respectively, form phenyl, or alkylene which together with -CH-R₁ or -CH-R₂, respectively, form benzocycloalkyl;
R₃ is hydrogen or methyl;
R₄ is cyclopentyl, α-naphthylmethyl, β-naphthylmethyl, benzyl or phenoxymethyl or alkoxymethyl, or R₃ and R₄ and the carbon and nitrogen atoms through which R₃ and R₄ are attached taken together form heterocyclyl; and
R' is hydrogen, methyl, ethyl, benzyl, cyclohexylmethyl or palmitoyl, provided that when R₃ and R₄ and the carbon and nitrogen atoms through which R₃ and R₄ are attached together form prolinyl, hydroxyprolinyl, methoxyprolinyl or thiazolidinyl, the 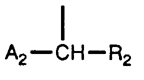
group attached to the 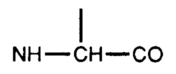
moiety together form alaninyl, leucinyl, valinyl or isoleucinyl and A₁ is hydrogen or methyl, then R₁ is not hydrogen, alkyl having 1 to 5 carbon atoms, unsubstituted phenyl or unsubstituted benzyl;
and the pharmaceutically acceptable salts thereof.

### DETAILED DESCRIPTION OF THE INVENTION

As used above, and throughout the description of the invention, the following terms, unless otherwise indicated, shall be understood to have the following meanings:

The "*" designation on the carbons in the compound according to the invention represents that the carbons are chiral.

"Alkyl" means a straight- or branched-chain hydrocarbon group having about 1 to about 8 carbon atoms in the chain. Preferred alkyl groups are methyl, ethyl, propyl *n*-butyl, *t*-butyl, *n*-pentyl or *n*-octyl.

"Acyl" means an alkyl-CO- group in which the alkyl group is as previously described. Preferred acyl have an alkyl containing about 1 to about 3 carbon atoms in the alkyl group. Exemplary groups are acetyl, propanoyl, 2-methylpropanoyl or butanoyl.

"Alkoxy" means an alkyl-O- group in which the alkyl group is as previously described. Exemplary alkoxy groups include methoxy, ethoxy, *n*-propoxy, *i*-propoxy, *n*-butoxy and heptoxy.

"Alkyloxymethyl" means an alkyl-O-CH₂- group in which the alkyl group is as previously described. When R₄ is alkyloxymethyl it is preferred that the alkyl contains about 1 to about 6 carbon atoms. Exemplary alkoxy groups include methoxymethyl, ethoxymethyl, *n*-propoxymethyl, *i*-propoxymethyl, *n*-butoxymethyl and heptoxymethyl.

"Alkylamino" means an amino group substituted by one or two alkyl groups as previously described. Exemplary alkylamino groups include methylamino, dimethylamino and diethylamino.

"Alkylene" means a bivalent hydrocarbon chain having from about 1 to about 6 carbon atoms. The most preferred alkylene groups are ethylene (-CH₂CH₂-) and propylene (-CH₂-)₃.

"Halogen" means fluoride, chloride or bromide.

"Heterocyclyl" means an about 4 to about 6 membered ring, optionally containing a second hetero atom, chosen from amongst N, O or S, and the heterocyclyl may be substituted. Representative heterocyclyl moieties include 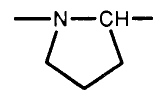
and 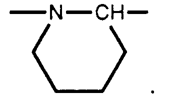
Preferred substituents of the heterocyclyl include hydroxyl, alkoxy containing about 1 to about 4 carbon atoms, trifluoromethyl, fluorine or a bis fused hydrocarbon chain radical of about 3 to about 4 carbons which radical when substituted on the heterocyclyl forms a saturated or unsaturated hydrocarbon ring having about 5 to about 6 members.

"Phenyl" is unsubstituted or may be substituted by hydroxy, alkoxy containing about 1 to about 4 carbon atoms, amino, alkylamino, trifluoromethyl or halogen.

"Benzyl" means phenyl-CH₂- wherein the phenyl is unsubstituted or may be substituted.

"Benzyloxy" means phenyl-CH₂-O- wherein the phenyl is unsubstituted or may be substituted.

"Benzoyl" means phenyl-CO- wherein the phenyl is unsubstituted or may be substituted.

The preferred compounds are described by the formula 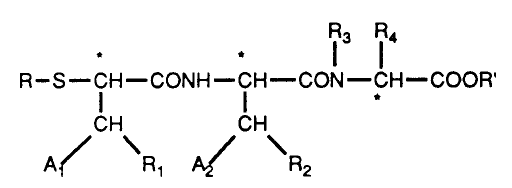
wherein:
R is hydrogen;
R₁ and R₂ are independently alkyl, alkoxy, phenyl, benzyl or benzyloxy;
A₁ and A₂ are independently hydrogen, methyl, or together with -CH-R₁ or -CH-R₂, respectively, form phenyl, or together with -CH-R₁ or -CH-R₂, respectively, form indanyl;
R₄ is benzyl in which the phenyl ring is optionally substituted by hydroxyl, or R₃ and R₄ and the carbon and nitrogen atoms through which R₃ and R₄ are attached taken together form a saturated 5 or 6 member heterocyclyl; and
R' is hydrogen.

The compounds of the present invention may be useful in the form of the free base or acid or in the form of a pharmaceutically acceptable salt thereof. All forms are within the scope of the invention.

Where the compound of the invention is substituted with an acidic moiety, base addition salts may be formed and are simply a more convenient form for use; and in practice, use of the salt form inherently amounts to use of the free acid form. The bases which can be used to prepare the base addition salts include preferably those which produce, when combined with the free acid, pharmaceutically acceptable salts, that is, salts whose cations are non-toxic to the animal organism in pharmaceutical doses of the salts, so that the beneficial mixed inhibition of NEP and ACE inherent in the compound of the invention is not vitiated by side effects ascribable to the cations. Although pharmaceutically acceptable salts of said acidic compounds are preferred, all base addition salts are useful as sources of the free acid form even if the particular salt, per se, is desired only as an intermediate product as for example, when the salt is formed only for purposes of purification, and identification, or when it is used as intermediate in preparing a pharmaceutically acceptable salt by ion exchange procedures. Pharmaceutically acceptable salts within the scope of the invention are those derived from the following bases: sodium hydroxide, potassium hydroxide, calcium hydroxide, aluminum hydroxide, lithium hydroxide, magnesium hydroxide, zinc hydroxide, ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, tetramethylammonium hydroxide, and the like.

Metal salts of compounds of the present invention may be obtained by contacting a hydroxide, carbonate or similar reactive compound of the chosen metal in an aqueous solvent with the free acid form of the compound. The aqueous solvent employed may be water or it may be a mixture of water with an organic solvent, preferably an alcohol such as methanol or ethanol, a ketone such as acetone, an aliphatic ether such as tetrahydrofuran, or an ester such as ethyl acetate. Such reactions are normally conducted at ambient temperature but they may, if desired, be conducted with heating.

Amine salts of compounds of the present invention may be obtained by contacting an amine in an aqueous solvent with the free acid form of the compound. Suitable aqueous solvents include water and mixtures of water with alcohols such as methanol or ethanol, ethers such as tetrahydrofuran, nitriles such as acetonitrile, or ketones such as acetone. Amino acid salts may be similarly prepared.

Preferred base addition salts have a cation selected from the group consisting of ammonium, sodium, calcium, protonated N-methyl-D-glucamine, protonated lysine, protonated arginine and protonated dicyclohexylamine.

Where the compound of the present invention is substituted with a basic moiety, acid addition salts may be formed and are simply a more convenient form for use; and in practice, use of the salt form inherently amounts to use of the free base form. The acids which can be used to prepare the acid addition salts include preferably those which produce, when combined with the free base, pharmaceutically acceptable salts, that is, salts whose anions are non-toxic to the animal organism in pharmaceutical doses of the salts, so that the beneficial mixed inhibition of NEP and ACE inherent in the compound of the invention is not vitiated by side effects ascribable to the anions. Pharmaceutically acceptable salts within the scope of the invention are those derived from the following acids: mineral acids such as hydrochloric acid, sulfuric acid, phosphoric acid and sulfamic acid; and organic acids such as acetic acid, citric acid, lactic acid, tartaric acid, malonic acid, methanesulfonic acid, ethanesulfonic acid, benzenesulfonic acid, p-toluenesulfonic acid, cyclohexylsulfamic acid, quinic acid, and the like. The corresponding acid addition salts comprise the following: hydrochloride, sulfate, phosphate, sulfamate, acetate, citrate, lactate, tartarate, malonate, methanesulfonate, ethanesulfonate, benzenesulfonate, p-toluenesulfonate, cyclohexylsulfamate and quinate, respectively.

The acid addition salts of the compounds of this invention are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

The compound according to the invention contains several asymmetric centers and may exist in the form of pure stereoisomers or in the form of mixtures of stereoisomers. Preferred compounds according to the invention are those wherein the carbon atoms of the principal chain marked by an asterisk (*) have the L configuration.

The present compounds may be prepared by the acylation of a dipeptide of the formula 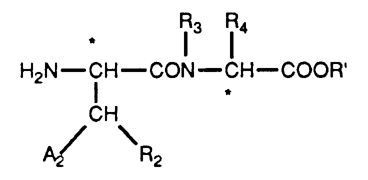
wherein
A₂, R₂, R₃, R₄ and R' are as defined above, by an acid of the formula 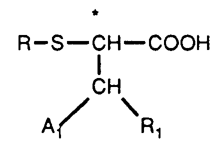
wherein
R, A₁ and R₁ are as defined above, under the usual conditions used in peptide chemistry and described, for example, by Bodansky et al., "Peptide Synthesis", J. Wiley and Sons Edit.

The acid reactant may be prepared from the corresponding α-amino acid by halogenating deamination according to Fischer, Ann., 357, I-24 (1907), followed by nucleophilic substitution of the halogen atom.

The present invention is further exemplified but not limited by the following illustrative examples.

### 1. PREPARATION OF THE INTERMEDIATES

### EXAMPLE A

### (R)-2-Bromo-3-phenylpropanoic Acid

Sodium nitrite (27 g in water) is added at 0°C to a solution of D-phenylalanine (40 g) in a mixture of 48% hydrobromic acid/water (1:1 by volume). The mixture is stirred for 30 minutes at 0°C and then for 2 hours 30 minutes at a temperature close to 20°C. The reaction mixture is extracted with ether. The organic extracts are washed with water and then with a saturated sodium chloride solution and then dried over sodium sulphate. After filtration and concentration to dryness, the obtained residue is purified by distillation. (R)-2-Bromo-3-phenylpropanoic acid (33 g) is obtained which has the following characteristics: B.p._{1 kPa} = 154°C; R_{f} = 0.47 (methylene chloride/methanol). The yield is 60%.

### EXAMPLE B

### (S)-2-Acetylthio-3-Phenylpropanoic Acid

Thioacetic acid (9.3 cc) and potassium carbonate (7.5 g) in water (150 cc) is added under nitrogen atmosphere to a solution of (R)-2-bromo-3-phenylpropanoic acid (25 g) in 1M sodium hydroxide (110 cc). The solution is stirred for 15 hours at a temperature close to 20°C. After evaporation under reduced pressure, the residue is taken up with water and ethyl acetate. The aqueous phase is separated, acidified to pH = 2 and then extracted with ethyl acetate. After washing, the organic phase is dried over sodium sulphate. After filtration and concentration under reduced pressure, (S)-2-acetylthio-3-phenylpropanoic acid (18.5 g) is obtained with a yield of 75% in the form of an oil having the following characteristics: R_{f} = 0.49 [hexane/ethyl acetate/acetic acid (6:4:0.5 by volume)].

### EXAMPLE C

### (R)-2-Bromo-3-Phenylpentanoic Acid

Working as in Example A, but starting from D-isoleucine, (R)-2-bromo-3-phenylpentanoic acid having the following characteristics is obtained with a yield of 74%: R_{f} = 0.38 [methylene chloride/methanol (1:1 by volume)].

### EXAMPLE D

### (S)-2-Acetylthio-3-Phenylpentanoic Acid

A solution of (R)-2-bromo-3-phenylpentanoic acid in dimethylformamide is added to a solution of thioacetic acid (1.5 equivalents) and sodium hydride (2.5 equivalents) in anhydrous dimethylformamide. The mixture is stirred for 3 hours at a temperature close to 20°C. After customary treatment, (S)-2-acetylthio-3-phenylpentanoic acid is obtained with a yield of 78% in the form of a yellow oil having the following characteristics: R_{f} = 0.44 [methylene chloride/methanol (9:1 by volume)].

### EXAMPLE E

### (R)-2-Bromo-4-Phenylbutanoic Acid

Working as in Example A, but starting from D-2-amino-4-phenylbutanoic acid, (R)-2-bromo-4-phenylbutanoic acid having the following characteristics is obtained with a yield of 72%: R_{f} = 0.30 [methylene chloride/methanol/acetic acid (10:0.1:0.2 by volume)].

### EXAMPLE F

### (S)-2-Acetylthio-4-Phenylbutanoic Acid

Working as in Example D but starting from (R)-2-bromo-4-phenylbutanoic acid, (S)-2-acetylthio-4-phenylbutanoic acid having the following characteristics is obtained with a yield of 80%: R_{f} = 0.35 [methylene chloride/methanol/acetic acid (9:0.3:0.1 by volume)].

### EXAMPLE G

### (R)-2-Bromo-3-Methylbutanoic Acid

Working as in Example A, but starting from D-valine, (R)-2-bromo-3-methylbutanoic acid is obtained with a yield of 69% in the form of an oil which crystallizes slowly having the following characteristics: R_{f} = 0.52 [methylene chloride/methanol/acetic acid (3:0.5:0.5 by volume)].

### EXAMPLE H

### (S)-2-Acetylthio-3-Methylbutanoic Acid

Working as in Example B, but starting from (R)-2-bromo-3-methylbutanoic acid, (S)-2-acetylthio-3-methylbutanoic acid is obtained with a yield of 94% in the form of an oil having the following characteristics: R_{f} = 0.28 [n-hexane/ethyl acetate (4:6 by volume)].

### EXAMPLE I

### (R)-2-Bromo-2-Phenylacetic Acid

Working as in Example A, but starting from D-phenylglycine, (R)-2-bromo-2-phenylacetic acid is obtained with a yield of 72% in the form of an oil which crystallizes slowly having the following characteristics: R_{f} = 0.63 [methylene chloride/methanol/acetic acid (9:1:0.5 by volume)].

### EXAMPLE J

### (S)-2-Acetylthio-2-Phenylacetic Acid

Working as in Example B, but starting from (R)-2-bromo-2-phenylacetic acid, (S)-2-acetylthio-2-phenylacetic acid is obtained with a yield of 74% in the form of an oil which crystallizes slowly having the following characteristics: R_{f} = 0.42 [n-hexane/ethyl acetate (4:6 by volume)].

### EXAMPLE K

### L-Ile-Tyr-OMe

1) A solution of methyl tyrosinate hydrochloride (1 equivalent) and triethylamine (1 equivalent) in chloroform, a solution of hydroxybenzotriazole (1 equivalent) in tetrahydrofuran and a solution of dicyclohexylcarbodiimide in chloroform are added successively at 0°C to a solution of t-butoxycarbonyl-L-isoleucine in tetrahydrofuran. The mixture is stirred for 1 hour at 0°C and then for 16 hours at a temperature close to 20°C. After customary treatment, the protected dipeptide (Boc-L-Ile-Tyr-OMe) having the following characteristics is obtained with a yield of 98%: R_{f} = 0.47 [methylene chloride/methanol (9:1 by volume)].
2) The protected dipeptide is dissolved in methylene chloride and then treated with 20 equivalents of trifluoroacetic acid. After 1 hour at 0°C and 2 hours at a temperature close to 20°C, the mixture is concentrated under reduced pressure. The residue is triturated with ether and then dried under reduced pressure. The dipeptide L-Ile-Tyr-OMe is thus obtained with a yield of 98% in the form of a white solid having the following characteristics: R_{f} = 0.23 [methylene chloride/methanol (9:1 by volume)].

### EXAMPLE L

### L-Val-Tyr-OMe

1) Working as in Example K-1), but starting from t-butoxycarbonyl-L-valine and methyl tyrosinate hydrochloride, the protected dipeptide (Boc-L-Val-Tyr-OMe) is obtained with a yield of 98% in the form of an oil having the following characteristics: R_{f} = 0.53 [methylene chloride/methanol (9:1 by volume)].
2) The elimination of the protective group is carried out under the conditions described in Example K-2). The dipeptide L-Val-Tyr-OMe is thus obtained with a yield of 98% in the form of a white solid having the following characteristics: R_{f} = 0.40 [methylene chloride/methanoinacetic acid (8:2:0-5 by volume)].

### EXAMPLE M

### L-Nle-Tyr-OMe

1) Working as in Example K-1), but starting from t-butoxycarbonyl-L-norleucine and methyl tyrosinate hydrochloride, the protected dipeptide Boc-L-Nle-Tyr-OMe is obtained with a yield of 99% in the form of an oil having the following characteristics: R_{f} = 0.67 [methylene chloride/methanol (9:1 by volume)].
2) The elimination of the protective group is carried out under the conditions described in Example K-2). The dipeptide L-Nle-Tyr-OMe is thus obtained with a yield of 70% in the form of a white solid having the following characteristics: R_{f} = 0.10 [methylene chloride/methanol (25:5 by volume)].

### EXAMPLE N

### L-Leu-Tyr-OMe

1) Working as in Example K-1), but starting from t-butoxycarbonyl-L-leucine and methyl tyrosinate hydrochloride, the protected dipeptide Boc-L-Leu-Tyr-OMe is obtained with a yield of 99% in the form of an oil having the following characteristics: R_{f} = 0.42 [methylene chloride/methanol (9:1 by volume)].
2) The elimination of the protective group is carried out under the conditions described in Example K-2). The dipeptide L-Leu-Tyr-OMe is thus obtained with a yield of 82% in the form of a white solid having the following characteristics: R_{f} = 0.11 [methylene chloride/methanol (9:1 by volume)].

### EXAMPLE O

### L-Phe-L-Tyr-OMe

1) Working as in Example K-1), but starting from t-butoxycarbonyl-L-phenylalanine and methyl L-tyrosinate hydrochloride, the protected dipeptide Boc-L-Phe-L-Tyr-OMe is obtained with a yield of 97% in the form of an oil having the following characteristics: R_{f} = 0.5 [methylene chloride/methanol (9:1 by volume)].
2) The elimination of the protective group is carried out under the conditions described in Example K-2). The dipeptide L-Phe-L-Tyr-OMe is thus obtained with a yield of 85% in the form of a white solid having the following characteristics: R_{f} = 0.25 [methylene chloride/methanoinacetic acid (9:1:05 by volume)].

### EXAMPLE P

### L-Ile-L-Pro-OMe

1) Working as in Example K-I), but starting from t-butoxycarbonyl-L-isoleucine and methyl L-prolinate, the protected dipeptide Boc-L-Ile-L-Pro-OMe is obtained with a yield of 92% in the form of an oil having the following characteristics: R_{f} = 0.44 [methylene chloride/methanol (9:0.5 by volume)].
2) The elimination of the protective group is carried out under the conditions described in Example K-2). The dipeptide L-Ile-L-Pro-OMe is thus obtained with a yield of 96% in the form of a white solid having the following characteristics: R_{f} = 0.37 [methylene chloride/methanol/acetic acid (9:1:0.5 by volume)].

### EXAMPLE Q

### L-Val-L-Pro-OMe

1) Working as in Example K-1), but starting from t-butoxycarbonyl-L-valine and methyl L-prolinate, the protected dipeptide Boc-L-Val-L-Pro-OMe is obtained with a yield of 98% in the form of an oil having the following characteristics: R_{f} = 0.42 [methylene chloride/methanol (9:0.5 by volume)].
2) The elimination of the protective group is carried out under the conditions described in Example K-2). The dipeptide L-Val-L-Pro-OMe is thus obtained with a yield of 86% in the form of a white solid having the following characteristics: R_{f} = 0.50 [methylene chloride/methanol/acetic acid (9:1:0.5 by volume)].

### EXAMPLE R

### L-Ile-L-Pip-OMe

1) Working as in Example K-I), but starting from t-butoxycarbonyl-L-isoleucine and L-2-methoxycarbonylpiperidine, the protected dipeptide Boc-L-Ile-L-Pip-OMe is obtained with a yield of 89% in the form of an oil having the following characteristics: R_{f} = 0.70 [methylene chloride/methanol/acetic acid (9:1:0.5 by volume)].
2) The elimination of the protective group is carried out under the conditions described in Example K-2). The dipeptide L-Ile-L-Pip-OMe is thus obtained with a yield of 79% in the form of a white solid having the following characteristics: R_{f} = 0.42 [methylene chloride/methanol/acetic acid (9:1:0.5 by volume)].

### 2. PREPARATION OF THE PRODUCT ACCORDING TO THE INVENTION

### EXAMPLE 1

### N-(2-mercapto-3-phenylpropanoyl)-Val-Tyr

Val-Tyr-OMe trifluoroacetate (1 equivalent) and triethylamine (1 equivalent) in chloroform and then a solution of 1-hydroxy-benzotriazole (1 equivalent) in tetrahydrofuran and a solution of dicyclohexylcarbodiimide (1 equivalent) in chloroform are added successively at 0°C to a solution of 2-acetylthio-3-phenylpropanoic acid (1 equivalent) in tetrahydrofuran. The mixture is stirred for 1 hour at 0°C and for 16 hours at a temperature close to 20°C. The precipitate is separated by filtration and the filtrate is concentrated to dryness under reduced pressure. The residue obtained is dissolved in ethyl acetate and then washed successively with a 10% citric acid solution (w/v), with water, with a 10% sodium carbonate solution (w/v), with water, then with a saturated sodium chloride solution. The organic phase is dried over sodium sulphate. After filtration and concentration to dryness, the residue is purified by chromatography. N-(2-Acetylthio-3-phenylpropanoyl)-Val-Tyr-OMe is thus obtained with a yield of 70%, in the form of an oily product. R_{f} = 0.54 [methylene chloride/methanol (9:0.5 by volume)].

The product obtained above is dissolved in degassed methanol and a 1M sodium hydroxide solution (3 equivalents) is added at 0°C. The mixture is stirred for 2 hours at a temperature close to 20°C and then a partition is carried out between degassed water and degassed ethyl acetate. The aqueous phase is acidified to pH = 2 and is then extracted with degassed ethyl acetate. The organic phase is washed with a saturated aqueous solution of sodium chloride, dried over sodium sulphate, filtered and then concentrated under reduced pressure. The residue obtained is chromatographed on silica gel, eluting with a methylene chloride/methanol/acetic acid mixture (9:0.5:0.5 by volume). N-(2-mercapto-3-phenylpropanoyl)-Val-Tyr is thus obtained with a yield of 65% in the form of a white solid having the following characteristics: high performance liquid chromatography: retention time: 8 minutes [acetonitrile/0.05% trifluoroacetic acid (45:55 by volume)]: proton nuclear magnetic resonance spectrum (deuterated dimethyl sulphoxide; chemical shifts in ppm): 0.8 [CH₃(Val)]; 1.90 [CHβ (Val)]; 2.60 (HS); 2.70, 2.80 and 2.05 [CH₂β (Tyr + Phe)]; 3.70 [CH α (Val)]; 4.20 (CH α (Phe + Tyr)]; 6.6 and 6.95 [Ar (Tyr)]; 7.8 [Ar (Phe)]; 8.05 and 8.20 (NH); 9.15 [OM (Tyr)]; 12.45 (COOH).

### EXAMPLE 2

### N-(2-Mercapto-3-Phenylpropanoyl)-Ile-Tyr-OMe

The coupling of 2-acetylthio-3-phenylpropanoic acid with the protected dipeptide Ile-Tyr-OMe is carried out under the conditions described in Example 1. N-(2-Acetylthio-3-phenylpropanoyl)-Ile-Tyr-OMe is obtained with a yield of 75% in the form of an oily product. R_{f} = 0.17 [hexane/ethyl acetate (5:5 by volume)].

Deprotection is carried out under the conditions of Example 1. N-(2-Mercapto-3-phenylpropanoyl)-Ile-Tyr-OMe is thus obtained with a yield of 70% in the form of a white solid melting at 185°C. R_{f} = 0.28 [methylene chloride/methanol/acetic acid (9:1:0.5 by volume)]; proton nuclear magnetic resonance spectrum (deuterated dimethyl sulphoxide; chemical shifts in ppm): 0.75 [CH₃ (Ile)], 1.1, 1.5 and 1.7 [CHβ CH₂γ (Ile)]; 2.70 (HS); 2.80, 2.90 and 3.15 [CH₂β (Phe + Tyr)]; 3.65, 4.15 and 4.30 [CH₂ α (Phe + Ile + Tyr)]; 6.60 and 7.00 [Ar (Tyr)]; 7.20 [Ar (Phe)]; 8.00 and 8.20 (NH); 12.20 (COOH).

### EXAMPLE 3

### N-(2-Mercapto-3-Phenylpropanoyl)-Ile-Pip

The coupling of 2-acetylthio-3-phenylpropanoic acid with the protected dipeptide Ile-Pip-OMe is carried out under the conditions described in Example 1. N-(2-Mercapto-3-phenylpropanoyl)-Ile-Pip-OMe is obtained with a yield of 51% in the form of an oily product. R_{f} = 0.30 [cyclohexane/ethyl acetate (5:5 by volume)].

Deprotection is carried out under the conditions described in Example 1. N-(2-Mercapto-3-phenylpropanoyl)-Ile-Pip is thus obtained with a yield of 65% in the form of a white solid. R_{f} = 0.48 [methylene chloride/methanol/acetic acid (9:0-5:0-25 by volume)]; proton nuclear magnetic resonance spectrum (deuterated dimethyl sulphoxide; chemical shifts in ppm): 0.80 [CH₃ (Ile)]; 1.02, 1.50 and 2.05 [CH₂β,γ andγ(Pip)]; 2.6 (HS); 2.80 and 3.05 [CH₂β (Phe)]; 3.7 [CH₂ε (Pip)]; 4.20, 4.70 and 4.95 (CH α; 7.20 [Ar (Phe)]; 8.20 and 8.40 (NH); 12.78 (COOH).

### EXAMPLE 4

### N-(2-Mercapto-3-Phenylpropanoyl)-Nle-Tyr

The coupling of 2-acetylthio-3-phenylpropanoic acid with the protected dipeptide Nle-Tyr-OMe is carried out under the conditions described in Example 1. N-(2-Acetylthio-3-phenylpropanoyl)-Nle-Tyr-OMe is thus obtained with a yield of 64% in the form of an oily product. R_{f} = 0.21 [hexane/ethyl acetate (6:4 by volume)].

Deprotection is carried out under the conditions described in Example 1. N-(2-Mercapto-3-phenylpropanoyl)-Nle-Tyr is thus obtained with a yield of 61% in the form of a white solid. R_{f} = 0.53 [methylene chloride/methanol (9:1 by volume)]; proton nuclear magnetic resonance spectrum (deuterated dimethyl sulphoxide; chemical shifts in ppm): 0.80 [CH₃ (Nle)]; 1.25 and 1.50 [CH₂β,γ and δ (Nle)]; 2.00 (HS); 2.75 and 2.95 [CH₂β (Phe + Tyr)]; 3.70 and 4.20 [CH α (Nle, Phe, Tyr)]; 6.60 and 6.95 [Ar (Tyr)]; 7.20 [Ar (Phe)]; 8.10 and 8.20 (NH); 9.15 [OM (Tyr)]; 12.3 (COOH); high performance liquid chromatography: retention time: 9.3 minutes [acetonitrile/0.05% trifluoroacetic acid (46:54 by volume)].

### EXAMPLE 5

### N-(2-Mercapto-3-Phenylpropanoyl)-Leu-Tyr

The coupling of 2-acetylthio-3-phenylpropanoic acid with the protected dipeptide Leu-Tyr-OMe is carried out under the conditions described in Example 1. N-(2-Acetylthio-3-phenylpropanoyl)-Leu-Tyr-OMe is thus obtained in the form of an oily product. R_{f} = 0.18 [cyclohexane/ethyl acetate (5:5 by volume)].

Deprotection is carried out under the conditions described in Example 1. N-(2-Mercapto-3-phenylpropionyl)-Leu-Tyr is thus obtained with a yield of 70% in the form of a white solid. R_{f} = 0.45 [methylene chloride/methanol/acetic acid (9:1:0.5 by volume)]; proton nuclear magnetic resonance spectrum (deuterated dimethyl sulphoxide; chemical shifts in ppm): 0.80 [CH₃ (Leu)]; 1.30 [CH₂β (Leu)]; 1.5 (CH₂γ (Leu)]; 2.60 (HS); 2.70, 2.80 and 3.00 [CH₂β (Phe + Tyr)]; 3.50 and 4.20 [CH₂ α (Phe, Leu, Tyr)]; 6.60 and 6.90 [Ar (Tyr)]; 7.20 [Ar (Phe)]; 7.90 and 8.05 (NH); 9.10 [OH (Tyr)]; 12.50 (COOH).

### EXAMPLE 6

### N-(2-Mercapto-3-Methylpentanoyl)-Phe-Tyr

The coupling of 2-acetylthio-3-methylpentanoic acid with the protected dipeptide Phe-Tyr-OMe is carried out under the conditions described in Example 1. N-(2-Acetylthio-3-methylpentanoyl)-Phe-Tyr-OMe is thus obtained with a yield of 78% in the form of an oily product. R_{f} = 0.14 [cyclohexane/ethyl acetate (6:4 by volume)].

Deprotection is carried out under the conditions described in Example 1. N-(2-Mercapto-3-methylpentanoyl)-Phe-Tyr melting at 120°C is thus obtained with a yield of 70%. R_{f} = 0.40 [methylene chloride/methanol/acetic acid (9:1:0.2 by volume)]; proton nuclear magnetic resonance spectrum (deuterated dimethyl sulphoxide; chemical shifts in ppm): 0.75 [CH₃ (Ile)]; 1.00, 1.20 and 1.60 [CH₂β, CH₂γ (Ile)]; 2.65 (HS); 2.70 and 2.90 [CH₂β (Phe + Tyr)]; 6.60 and 6.95 [Ar (Tyr)]; 7.13 [Ar (Phe)]; 8.00 and 8.05 (NH); 9.10 [OH (Tyr)]; 12.6 (COOH).

### EXAMPLE 7

### N-(2-Mercapto-3-Methylpentanoyl)-Val-Tyr

The coupling of 2-acetylthio-3-methylpentanoic acid with the protected dipeptide Val-Tyr-OMe is carried out under the conditions described in Example 1. N-(2-Acetylthio-3-methylpentanoyl)-Val-Tyr-OMe is thus obtained with a yield of 78% in the form of an oily product. R_{f} = 0.41 [methylene chloride/methanol (9:5:0.5 by volume)].

Deprotection is carried out under the conditions described in Example 1. N-(2-Mercapto-3-methylpentanoyl)-Val-Tyr is thus obtained with a yield of 72% in the form of a white solid melting at 150°C. R_{f} = 0.20 [methylene chloride/methanol/acetic acid (9:0.5:0.25 by volume)]; proton nuclear magnetic resonance spectrum (deuterated dimethyl sulphoxide; chemical shifts in ppm): 0.80 [CH₃ (Val + Ile)]; 1.00 and 1.25 [CH₂γ (Ile)]; 1.65 [CHβ (Ile)]; 1.90 [CHβ (Val)]; 2.30 (HS); 2.70 and 2.85 [CH₂β (Tyr)]; 3.30 [CHα (Ile)]; 4.20 and 4.32 [CH α(Val and Tyr)]; 6.68 and 6.95 [Ar (Tyr)]; 7.90 and 8.10 [NH (Val and Tyr)]; 9.20 [OH (Tyr)]; 12.56 (COOH).

### EXAMPLE 8

### N-(2-Mercapto-4-Phenylbutanoyl)-Val-Tyr

The coupling of 2-acetylthio-4-phenylbutanoic acid with the protected dipeptide Val-Tyr-OCH₃ is carried out under the conditions described in Example 1. N-(2-Acetylthio-4-phenylbutanoyl)-Val-Tyr-OCH₃ is thus obtained with a yield of 82% in the form of an oily product. R_{f} = 0.49 [cyclohexane/ethyl acetate (5:5 by volume)].

Deprotection is carried out under the conditions described in Example 1. N-(2-Mercapto-4-phenylbutanoyl)-Val-Tyr is thus obtained with a yield of 98% in the form of a white product melting at 105°C. R_{f} = 0.65 [methylene chloride/methanol/acetic acid (9:1:0.5 by volume)]; high performance liquid chromatography: retention time: 9.10 minutes[acetonitrile/0.05% trifluoroacetic acid (45:55 by volume)]; proton nuclear magnetic resonance spectrum (deuterated dimethyl sulphoxide; chemical shifts in ppm): 0.75 [CH₃ (Val)]; 1.75 [CHβ (Val)]; 1.95 [CH₂γ(Phe)]; 2.50 [CH₂β (Phe)]; 2.72 and 2.90 [CH₂β (Tyr)]; 3.45 [CH α (Phe)]; 4.20 and 4.30 [CH α (Tyr and Val)]; 6.60 and 7.00 [Ar (Tyr)]; 7.12 and 7.20 [Ar (Phe)]; 7.20 and 8.10 (NH); 9.15 [OH (Tyr)]; 12.35 (COOH).

### EXAMPLE 9

### N-(2-Mercapto-3-Methylbutanoyl)-Ile-Tyr

The coupling of (S)-2-acetylthio-3-methylbutanoic acid with the protected dipeptide Ile-Tyr-OCH₃ is carried out under the conditions described in Example 1. -(2-Acetylthio-3-methylbutanoyl)-Ile-Tyr-OCH₃ is obtained with a yield of 85% in the form of an oily product. R_{f} = 0.28 [n-hexane/ethyl acetate (4:6 by volume)].

Deprotection is carried out under the conditions described in Example 1. N-(2-Mercapto-3-methylbutanoyl)-Ile-Tyr is obtained with a yield of 89% in the form of a white product melting at 199°C. R_{f} = 0.42 [methylene chloride/methanol/acetic acid (9:1:0.5 by volume)]; proton nuclear magnetic resonance spectrum (deuterated dimethyl sulphoxide; chemical shifts in ppm): 0.75 and 0.90 [CH₃ (Val and Ile)]; 1 [CHβ (Ile)]; 1.40 and 1.65 [CH₂γ (Ile)]; 1.80 [CHβ (Val)]; 2.30 (HS); 2.75 and 2.89 [CH₂β (Tyr)]; 3.15 [CH (SH)]; 4.15 [CH α (Ile)]; 4.32 [CH α (Tyr); 6.55 and 6.95 [Ar (Tyr)]; 7.9 and 8.10 (NH); 9.12 [OH (Tyr)]; 12.10 (COOH); high performance liquid chromatography: retention time: 5.84 minutes [acetonitrile/0.05% trifluoroacetic acid (5:5 by volume)].

### EXAMPLE 10

### N-(2-Mercapto-2-Phenylacetyl)-Ile-Tyr

The coupling of (S)-2-acetylthio-2-phenylacetic acid with the protected dipeptide Ile-Tyr-OCH₃ is carried out under the conditions described in Example 1. N-(2-Acetylthio-2-phenylacetyl)-Ile-Tyr-OCH₃ is obtained with a yield of 46% in the form on an oily product. R_{f} = 0.42 [n-hexane/ethylacetate (4:6 by volume)].

Deprotection is carried out under the conditions described in Example 1. N-(2-Mercapto-2-phenylacetyl)-Ile-Tyr is obtained with a yield of 73% in the form of an oil. R_{f} = 0.42 [methylene chloride/methanol/acetic acid (9:1:0.5 by volume)]; proton nuclear magnetic resonance spectrum (deuterated dimethyl sulphoxide; chemical shifts in ppm): 0.61 and 0.72 [CH₃ (Ile)]; 1.1 [CHβ (Ile)]; 1.4 and 1.7 [CH₂γ (Ile)]; 2.32 (HS); centered on 2.80 [CH₂β (Tyr)]; 3.3 [CH (SH)]; 4.20 [CH₂ α (Tyr)]; 4.82 [CH α (Ile)]; 6.55 and 6.95 [Ar (Tyr)]; centered on 7.30 [Ar (Phe)]; 8.15 [NH (Ile and Tyr)]; 9.10 [OH (Tyr)]; 12.45 (COOH).

### EXAMPLES 11 TO 35

Working as in Example 1, the products which are collected in Table 1 are obtained.

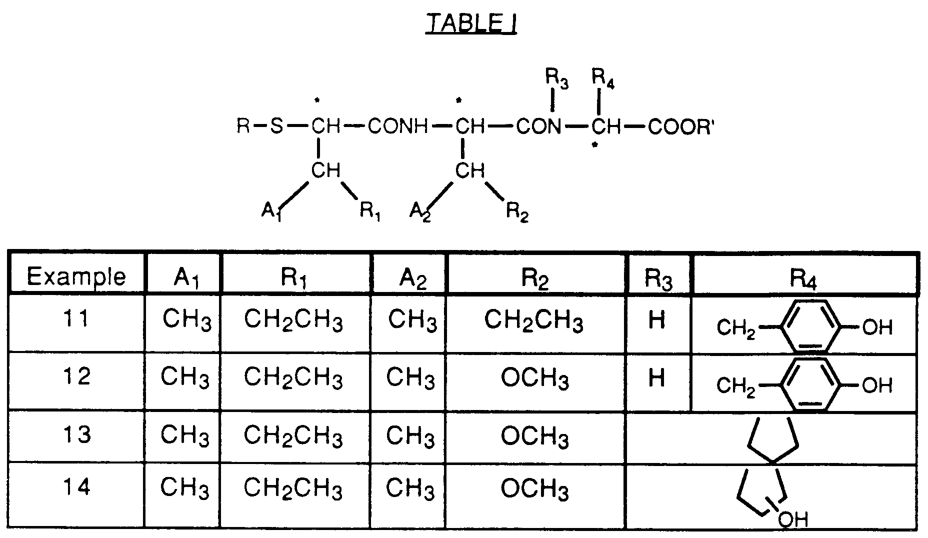

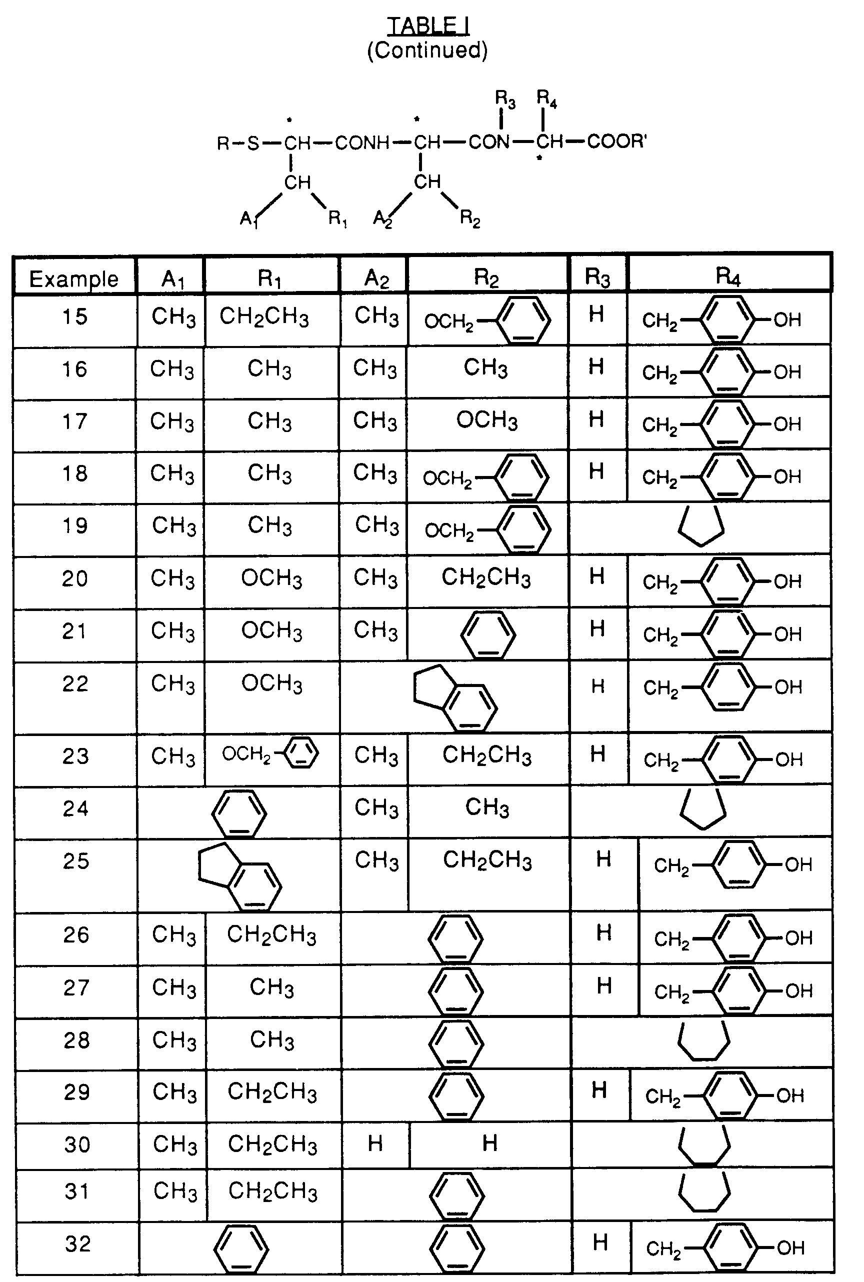

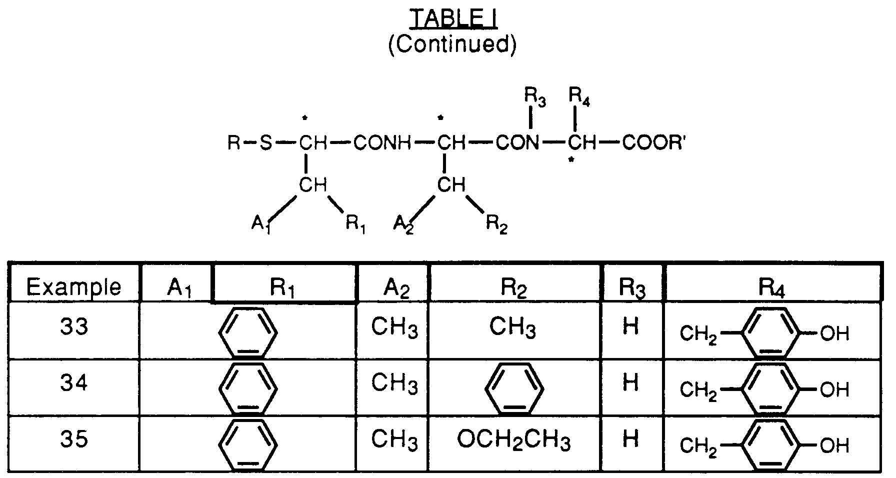

Compounds within the scope of the present invention, when administered to mammals, are potent antihypertensives due to their double inhibitory action on NEP and ACE. These compounds are capable of blocking the increase in the blood pressure caused by an increase in the vascular resistance and the blood volume due to the formation of angiotensin II from angiotensin I since ACE inhibitors block the production of angiotensin II. In addition, these compounds regulate auricular natriuretic peptide which is implicated in the regulation of arterial pressure. This peptide is liberated by the heart, endowed with a vasodilatory property and capable of controlling diuresis and natriuresis. Auricular natriuretic peptide is inactivated by NEP in the peripheral tissues. By inhibiting NEP with the compound according to the invention, a significant augmentation in diuresis and natriuresis in man is induced without causing the appearance of an increase in the amounts of renin and aldosterone that is usually found with the diuretics generally used in association with ACE inhibitors. Therefore, the compound according to the invention, i.e., the mixed inhibitors of NEP and ACE, may be used to alleviate hypertension of various origins without the co-administration of other diuretics.

Thus compounds according to the present invention are effective in the treatment of congestive heart failures and various types of hypertension, in particular hypertension connected with an increase in the blood volume. Accordingly, the hypertensive properties exhibited by the present compounds is preferred to the effects obtained with the concomitant administration of either or both of NEP and ACE inhibitors.

The enzymatic inhibitory properties of compounds according to the present invention is measured using [³H]D-Ala²-Leu-enkephalin as a substrate for neutral endopeptidase as described previously by Llorens et al., Biochem. Biophys. Res. Commun., 96, 1710 (1980) and Z-Phe-His-Leu in the case of peptidyldipeptidase A according to the process described in Biochem. Biophys. Acta, 206, 136-142 (1970).

The antihypertensive properties of the present compounds are determined in rat models including that of rat hypertension induced by DOCA salt and the spontaneously hypertensive male rat (SHR) according to Trapani et al., J. Cardiovasc. Pharmacol., 14, 419-424 (1989).

Table II below presents selected pharmacological data for the most preferred compounds within the scope of the present invention.

**TABLE II**

| Inhibitory Effect on NEP and ACE | | |
|---|---|---|
| Examples | IC₅₀ (nM) NEP | IC₅₀ (nM) ACE |
| 1 | 5±2 | 4±1 |
| 2 | 3.2±0.3 | 2.5±0.1 |
| 3 | 5±2 | 2±1 |
| 4 | 18±2 | 30±5 |
| 5 | 3±2 | 30±5 |
| 6 | 3±1 | 8±2 |
| 7 | 5±2 | 2±0.5 |
| 8 | 10±1 | 8±2 |
| 9 | 1.4±0.2 | 0.3±0.1 |
| 10 | 4.5±0.5 | 3.5±0.5 |

The compounds of this invention normally may be administered orally or parenterally, in the treatment of patients suffering from hypertension. As used herein, the term "patients" includes human and other mammals.

The compounds of the present invention, in the pure state in the form of the free base or salt, may be formulated for administration in any convenient way, and the invention includes within its scope pharmaceutical compositions containing at least one compound according to the invention adapted for use in human or veterinary medicine. Such compositions may be formulated in a conventional manner using one or more pharmaceutically acceptable carriers or excipients. Suitable carriers include adjuvants, diluents or fillers, sterile aqueous media and various non toxic organic solvents. The compositions may be formulated in the form of tablets, capsules, lozenges, troches, hard candies, powders, granules, aqueous suspensions, or solutions, injectable solutions, elixirs, syrups and the like and may contain one or more agents selected from the group including wetting agents, thickening agents, stabilizing agents, aromatic agents, sweetening agents, flavoring agents, coloring agents, coating agents, glazes and preserving agents, in order to provide a pharmaceutically acceptable preparation

The particular carrier and the ratio of the active ingredient to carrier are determined by the solubility and chemical properties of the compounds, the particular mode of administration and standard pharmaceutical practice. For example, excipients such as lactose, sodium citrate, calcium carbonate and dicalcium phosphate and various disintegratants such as starch, cellulose, alginic acid and certain complex silicates, together with lubricating agents such as magnesium stearate, sodium lauryl sulphate and talc, can be used in producing tablets. For a capsule form, sucrose, lactose and high molecular weight polyethylene glycols are among the preferred pharmaceutically acceptable carriers. Where aqueous suspensions for oral use are formulated, the carrier can be emulsifying or suspending agents. Diluents such as ethanol, propylene glycol, glycerin and chloroform and their combinations can be employed as well as other materials.

For parental administration, solutions or suspensions of these compounds in a vegetable oil, such as, in particular olive, sesame or peanut oil, aqueous propylene glycol solutions, injectable organic esters such as ethyl oleate or other compatible organic solvents as well as sterile aqueous solutions of the soluble pharmaceutically acceptable salts described herein can be employed. Solutions of the salts of these compounds are especially suited for administration by intramuscular and subcutaneous injection. The aqueous solutions, including those of the salts dissolved in pure distilled water, are suitable for administration by intravenous injection, provided that their pH is properly adjusted, and that they are suitably buffered, made isotonic with sufficient saline or glucose. Sterilization may be carried out in several ways, such a by sterile filtration, e.g., microfiltration, incorporation of sterilizing agents, irradiation or heating. Sterile solid compositions may be dissolved in a sterile injectable medium.

The dosage regimen used in carrying out the methods of this invention is that which insures maximum therapeutic response until improvement is obtained and thereafter the minimum effective level which gives relief. Thus, in general, the dosages are those that are therapeutically effective in lowering blood pressure in the treatment of hypertension. In general, the oral dose may be between about 0.1 and about 100 (preferably in the range of 1 to 10 mg/kg), and the iv. dose about 0.01 to about 10 mg/kg (preferably in the range of 0.1 to 5 mg/kg), bearing in mind, of course, that in selecting the appropriate dosage in any specific case, consideration must be given to the patient's weight, general health, age and other factors which may influence response to the drug.

The compounds of this invention may be administered as frequently as is necessary to achieve and sustain the desired therapeutic response. Some patients may respond quickly to a relatively large or small dose and require lesser amounts as a maintenance dosage. On the other hand, other patients may require sustained dosing from about 1 to about 4 times a day depending on the physiological needs of the particular patient. Usually the drug may be administered orally about 1 to about 4 times per day. It is anticipated that many patients will require no more than about one to about two doses daily.

It is also anticipated that the present invention would be useful as an injectable dosage form which may be administered in an emergency to a patient suffering acute hypertension. Such treatment may be followed by intravenous infusion of the active compound and the amount of compound infused into such a patient should be effective to achieve and maintain the desired therapeutic response.

## Claims

1. A compound of the formula 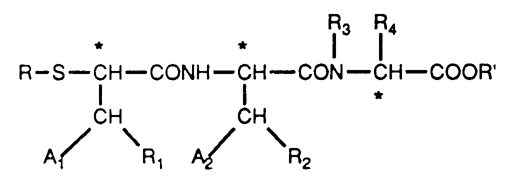 wherein:
R is hydrogen, acyl, benzoyl, adamantoyl or a radical of the formula 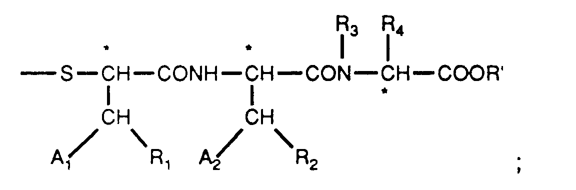 R₁ and R₂ are independently hydrogen, alkyl, phenyl, benzyl, alkoxy, alkyloxymethyl or benzyloxy;
A₁ and A₂ are independently hydrogen, methyl, or together with -CH-R₁ or -CH-R₂, respectively, form phenyl, or alkylene which together with -CH-R₁ or -CH-R₂, respectively, form benzocycloalkyl;
R₃ is hydrogen or methyl;
R₄ is cyclopentyl, α-naphthylmethyl, β-naphthylmethyl, benzyl or phenoxymethyl or alkoxymethyl, or R₃ and R₄ and the carbon and nitrogen atoms through which R₃ and R₄ are attached taken together form heterocyclyl; and
R' is hydrogen, methyl, ethyl, benzyl, cyclohexylmethyl or palmitoyl, provided that when R₃ and R₄ and the carbon and nitrogen atoms through which R₃ and R₄ are attached together form prolinyl, hydroxyprolinyl, methoxyprolinyl or thiazolidinyl, the 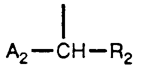 group attached to the 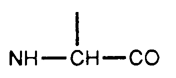 moiety together form alaninyl, leucinyl, valinyl or isoleucinyl and A₁ is hydrogen or methyl, then R₁ is not hydrogen, alkyl having 1 to 5 carbon atoms, unsubstituted phenyl or unsubstituted benzyl;
or a pharmaceutically acceptable salt thereof.

2. The compound according to claim 1 wherein:
R is hydrogen;
R₁ and R₂ are independently alkyl, alkoxy, phenyl, benzyl or benzyloxy;
A₁ and A₂ are independently hydrogen, methyl, or together with -CH-R₁ or-CH-R₂, respectively, form phenyl, or together with -CH-R₁ or -CH-R₂, respectively, form indanyl;
R₄ is benzyl in which the phenyl ring is optionally substituted by hydroxyl, or R₃ and R₄ and the carbon and nitrogen atoms through which R₃ and R₄ are attached taken together form a saturated 5 or 6 member heterocyclyl; and
R' is hydrogen.

3. A process for the preparation of a compound of claim 1, comprising acylating a dipeptide of the formula 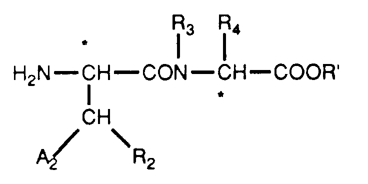 with an acid of the formula

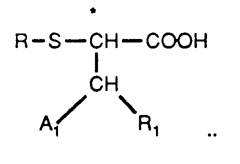

4. A process for the preparation of a compound according to claim 2 comprising acylating a dipeptide of the formula: 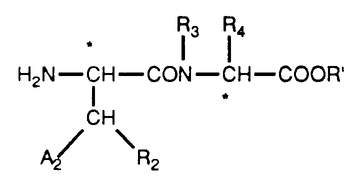 with an acid of the formula

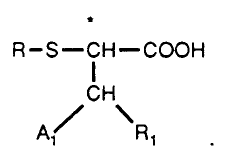

5. A pharmaceutical composition comprising a compound of claim 1 and a pharmaceutically acceptable carrier.

6. A pharmaceutical composition comprising a compound of claim 2 and a pharmaceutically acceptable carrier.

7. The compound of claim 1 selected from the group consisting of:
N-[N'-(2-mercapto-3-phenylpropanoyl)valyl]tyrosine;
N-[N'-(2-mercapto-3-phenylpropanoyl)isoleucinyl]tyrosine;
N-[N'-(2-mercapto-3-phenylpropanoyl)isoleucinyl]piperidin-2-oic acid;
N-[N'-(2-mercapto-3-phenylpropanoyl)n-leucinyl]tyrosine;
N-[N'-(2-mercapto-3-phenylpropanoyl)leucinyl]tyrosine;
N-[N'-(2-mercapto-3-methylpentanoyl)phenalanyl]tyrosine;
N-[N'-(2-mercapto-3-methylpentanoyl)valyl]tyrosine;
N-[N'-(2-mercapto-4-phenylbutanoyl)valyl]tyrosine;
N-[N'-(2-mercapto-3-methylbutanoyl)isoleucinyl]tyrosine;
N-[N'-(2-mercapto-2-phenylacetyl)isoleucinyl]tyrosine;
N-[N'-(2-mercapto-3-methylpentanoyl)isoleucinyl]tyrosine;
N-[N'-(2-mercapto-3-methylpentanoyl)-2-amino-3-methoxybutanoyl]tyrosine;
N-[N'-(2-mercapto-3-methylpentanoyl)-2-amino-3-methoxybutanoyl]proline;
N-[N'-(2-mercapto-3-methylpentanoyl)-2-amino-3-methoxybutanoyl]hydroxyproline;
N-[N'-(2-mercapto-3-methylpentanoyl)-2-amino-3-benzyloxybutanoyl]tyrosine;
N-[N'-(2-mercapto-3-methylbutanoyl)-2-amino-3-methylbutanoyl]tyrosine;
N-[N'-(2-mercapto-3-methylpentanoyl)-2-amino-3-methoxybutanoyl]tyrosine;
N-[N'-(2-mercapto-3-methylbutanoyl)-2-amino-3-benzyloxybutanoyl]tyrosine;
N-[N'-(2-mercapto-3-methylbutanoyl)-2-amino-3-benzyloxybutanoyl]proline;
N-[N'-(2-mercapto-3-methoxybutanoyl)isoleucinyl]tyrosine;
N-[N'-(2-mercapto-3-methoxybutanoyl)-3-methylphenalanyl]tyrosine;
N-[N'-(2-mercapto-3-methoxybutanoyl)-2-amino-2-indan-2'-ylacetyl]tyrosine;
N-[N'-(2-mercapto-3-benzyloxybutanoyl)isoleucinyl]tyrosine;
N-[N'-(2-mercapto-2-phenylacetyl)valinyl]tyrosine;
N-[N'-(2-mercapto-2-indan-2'-yl)isoleucinyl]tyrosine;
N-[N'-(2-mercapto-3-methylpentanoyl)-2-amino-2-phenylacetyl]tyrosine;
N-[N'-(2-mercapto-3-methylbutanoyl)-2-amino-2-phenylacetyl]tyrosine;
N-[N'-(2-mercapto-3-methylbutanoyl)-2-amino-2-phenylacetyl]piperidin-2-oic acid;
N-[N'-(2-mercapto-3-methylpentanoyl)-2-amino-2-phenylacetyl]tyrosine;
N-[N'-(2-mercapto-3-methylpentanoyl)alanyl]piperidin-2-oic acid;
N-[N'-(2-mercapto-3-methylbutanoyl)-2-amino-2-phenylacetyl]piperidin-2-oic acid;
N-[N'-(2-mercapto-2-phenylacetyl)-2-amino-2-phenylacetyl]tyrosine;
N-[N'-(2-mercapto-2-phenylacetyl)valinyl]tyrosine;
N-[N'-(2-mercapto-2-phenylacetyl)-2-amino-3-phenylbutanoyl]tyrosine; and
N-[N'-(2-mercapto-2-phenylacetyl)-2-amino-3-ethoxybutanoyl]tyrosine.

8. The compound of claim 1 wherein the configuration at each of the asterisked carbons is L.

9. The method of treating hypertension in a mammal comprising administering an effective antihypertensive amount of a compound of claim 1.
